# EUROPEAN PATENT SPECIFICATION

(11) **EP 0 753 061 B1**
(45) Date of publication and mention of the grant of the patent: **15.06.2005**
(21) Application number: 95910622.0
(22) Date of filing: 06.03.1995
(51) Int. Cl.: C12N 15/10, C12N 15/63, C12N 1/21, A61K 39/02, C12N 1/20

(54) **ORAL VACCINE COMPOSITIONS AGAINST INFECTIONS WITH YERSINIA PESTIS**
ORALE IMPFSTOFFE GEGEN YERSINIA PESTIS INFEKTIONEN
COMPOSITIONS VACCINALES ORALES CONTRE LES INFECTIONS A YERSINIA PESTIS

(30) Priority: 08.03.1994 GB 9404577
(43) Date of publication of application: 15.01.1997
(73) Proprietor: The Secretary of State for Defence, Salisbury Wiltshire SP4 0JQ (GB)
(72) Inventor: TITBALL, Richard William, Wiltshire SP4 0JQ (GB); WILLIAMSON, Ethel Diane, Salisbury Wiltshire SP4 0JQ (GB); LEARY, Sophie Emma Clare, Salisbury Wiltshire SP4 0JQ (GB)
(74) Representative: Beckham, Robert William
(86) International application number: PCT/GB1995/000481
(87) International publication number: WO 1995/024475

(56) References cited:
- INFECTION AND IMMUNITY, vol. 62, no. 10, 1994 pages 4192-4201, V.L. MOTIN ET AL. 'Passive immunity to yersiniae mediated by anti-recombinant V antigen and protein A-V antigen fusion peptide'
- CONTRIB. MICROBIOL. IMMUNOL., vol. 12, 1991 pages 225-229, K. SATO ET AL. 'Preparation of monoclonal antibody to V antigen from Y. pestis'
- J. BACTERIOL., vol. 173, no. 8, 1991 pages 2649-2657, S.B. PRICE ET AL. 'The Y. pestis V antigen is a regulatory protein necessary for Ca-dependant growth and maximal expression of low Ca response virulence genes'
- J. BACTERIOL., vol. 171, no. 10, 1989 pages 5646-5653, S.B. PRICE ET AL. 'Molecular analysis of lcrGVH, the V antigen operon of Y. pestis'
- CHEN T.H.; ELBERG S.S.; EISLER D.M.: "Immunity in plague: protection of the vervet (Cercopithecus aethips) against pneumonic plague by the oral administration of live attenuated Yersinia pestis." JOURNAL OF INFECTIOUS DISEASES, vol. 135, no. 2, February 1977 (1977-02), pages 289-293,

### SEQUENCE LISTING

(1) GENERAL INFORMATION:
   (i) APPLICANT:
      (A) NAME: THE SECRETARY OF STATE FOR DEFENCE IN HER BRITANNIC MAJESTY
      (B) STREET: WHITEHALL
      (C) CITY: LONDON
      (E) COUNTRY: UNITED KINGDOM (GB)
      (F) POSTAL CODE (ZIP): SW1A 2HB

      (A) NAME: RICHARD WILLIAM TITBALL
      (B) STREET: DMD CBDE PORTON DOWN
      (C) CITY: SALISBURY
      (D) STATE: WILTSHIRE
      (E) COUNTRY: UNITED KINGDOM (GB)
      (F) POSTAL CODE (ZIP): SP4 OJQ

      (A) NAME: EDITH DIANE WILLIAMSON
      (B) STREET: DMD CBDE PORTON DOWN
      (C) CITY: SALISBURY
      (D) STATE: WILTSHIRE
      (E) COUNTRY: UNITED KINGDOM (GB)
      (F) POSTAL CODE (ZIP): SP4 OJQ

      (A) NAME: SOPHIE E C LEARY
      (B) STREET: DMD CBDE PORTON DOWN
      (C) CITY: SALISBURY
      (D) STATE: WILTSHIRE
      (E) COUNTRY: UNITED KINGDOM (GB)
      (F) POSTAL CODE (ZIP): SP4 OJQ
   (ii) TITLE OF INVENTION: VACCINES
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: C-DOS/MS-DOS
      (D) SOFTWARE: PatentIn Release @1.0. Version @1.25 (EPO)
(2) INFORMATION FOR SEQ ID NO: 1:
   (i) SEQUENCE CHARACTERISTICS:
      (A) LENGTH: 1014 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Yersinia pestis
   (ix) FEATURE:
      (A) NAME/KEY: CDS
      (B) LOCATION: 1..987 (xi)
   SEQUENCE DESCRIPTION: SEQ ID NO: 1:
(2) INFORMATION FOR SEQ ID NO: 2:
   (i) SEQUENCE CHARACTERISTICS:
      (A) LENGTH: 329 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Ile Ser Glu Phe Ile Arg Ala Tyr Glu Gln Asn Pro Gln His Phe Ile
   1               5                   10                  15
   Glu Asp Leu Glu Lys Val Arg Val Glu Gln Leu Thr Gly His Gly Ser
               20                  25                  30
   Ser Val Leu Glu Glu Leu Val Gln Leu Val Lys Asp Lys Asn Ile Asp
           35                  40                  45
   Ile Ser Ile Lys Tyr Asp Pro Arg Lys Asp Ser Glu Val Phe Ala Asn
       50                  55                  60
   Arg Val Ile Thr Asp Asp Ile Glu Leu Leu Lys Lys Ile Leu Ala Tyr
   65                  70                  75                  80
   Phe Leu Pro Glu Asp Ala Ile Leu Lys Gly Gly His Tyr Asp Asn Gln
                   85                  90                  95
   Leu Gln Asn Gly Ile Lys Arg Val Lys Glu Phe Leu Glu Ser Ser Pro
               100                 105                 110
   Asn Thr Gln Trp Glu Leu Arg Ala Phe Met Ala Val Met His Phe Ser
   115 120 125
   Leu Thr Ala Asp Arg Ile Asp Asp Asp Ile Leu Lys Val Ile Val Asp
   130 135 140
   Ser Met Asn His His Gly Asp Ala Arg Ser Lys Leu Arg Glu Glu Leu
   145 150 155 160
   Ala Glu Leu Thr Ala Glu Leu Lys Ile Tyr Ser Val Ile Gln Ala Glu
   165 170 175
   Ile Asn Lys His Leu Ser Ser Ser Gly Thr Ile Asn Ile His Asp Lys
   180 185 190
   Ser Ile Asn Leu Met Asp Lys Asn Leu Tyr Gly Tyr Thr Asp Glu Glu
   195 200 205
   Ile Phe Lys Ala Ser Ala Glu Tyr Lys Ile Leu Glu Lys Met Pro Gln
   210 215 220
   Thr Thr Ile Gln Val Asp Gly Ser Glu Lys Lys Ile Val Ser Ile Lys
   225 230 235 240
   Asp Phe Leu Gly Ser Glu Asn Lys Arg Thr Gly Ala Leu Gly Asn Leu
   245 250 255
   Lys Asn Ser Tyr Ser Tyr Asn Lys Asp Asn Asn Glu Leu Ser His Phe
   260 265 270
   Ala Thr Thr Cys Ser Asp Lys Ser Arg Pro Leu Asn Asp Leu Val Ser
   275 280 285
   Gln Lys Thr Thr Gln Leu Ser Asp Ile Thr Ser Arg Phe Asn Ser Ala
   290 295 300
   Ile Glu Ala Leu Asn Arg Phe Ile Gln Lys Tyr Asp Ser Val Met Gln
   305 310 315 320
   Arg Leu Leu Asp Asp Thr Ser Gly Lys
   325
(2) INFORMATION FOR SEQ ID NO: 3:
   (i) SEQUENCE CHARACTERISTICS:
      (A) LENGTH: 1014 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Yersinia pestis
   (ix) FEATURE:
      (A) NAME/KEY: CDS
      (B) LOCATION: 1..987
   (xi) SEQUENCE DESCRIPTION: SEQ ID NO: 3:
   GGG ATC CCC GGA ATT CGA GCC TAC GAA CAA AAC CCA CAA CAT TTT ATT 48
   Gly Ile Pro Gly Ile Arg Ala Tyr Glu Gln Asn Pro Gln His Phe Ile
     1               5                  10                  15
   GAG GAT CTA GAA AAA GTT AGG GTG GAA CAA CTT ACT GGT CAT GGT TCT 96
   Glu Asp Leu Glu Lys Val Arg Val Glu Gln Leu Thr Gly His Gly Ser
                20                  25                  30
   TCA GTT TTA GAA GAA TTG GTT CAG TTA GTC AAA GAT AAA AAT ATA GAT 144
   Ser Val Leu Glu Glu Leu Val Gln Leu Val Lys Asp Lys Asn Ile Asp
            35                  40                  45
   ATT TCC ATT AAA TAT GAT CCC AGA AAA GAT TCG GAG GTT TTT GCC AAT 192
   Ile Ser Ile Lys Tyr Asp Pro Arg Lys Asp Ser Glu Val Phe Ala Asn
        50                  55                  60
   AGA GTA ATT ACT GAT GAT ATC GAA TTG CTC AAG AAA ATC CTA GCT TAT 240
   Arg Val Ile Thr Asp Asp Ile Glu Leu Leu Lys Lys Ile Leu Ala Tyr
    65                  70                  75                  80
   TTT CTA CCC GAG GAT GCC ATT CTT AAA GGC GGT CAT TAT GAC AAC CAA 288
   Phe Leu Pro Glu Asp Ala Ile Leu Lys Gly Gly His Tyr Asp Asn Gln
                    85                  90                  95
   CTG CAA AAT GGC ATC AAG CGA GTA AAA GAG TTC CTT GAA TCA TCG CCG 336
   Leu Gln Asn Gly Ile Lys Arg Val Lys Glu Phe Leu Glu Ser Ser Pro
               100                 105                 110
   AAT ACA CAA TGG GAA TTG CGG GCG TTC ATG GCA GTA ATG CAT TTC TCT 384
   Asn Thr Gln Trp Glu Leu Arg Ala Phe Met Ala Val Met His Phe Ser
           115                 120                 125
   TTA ACC GCC GAT CGT ATC GAT GAT GAT ATT TTG AAA GTG ATT GTT GAT 432
   Leu Thr Ala Asp Arg Ile Asp Asp Asp Ile Leu Lys Val Ile Val Asp
       130                 135                 140
   TCA ATG AAT CAT CAT GGT GAT GCC CGT AGC AAG TTG CGT GAA GAA TTA  480
   Ser Met Asn His His Gly Asp Ala Arg Ser Lys Leu Arg Glu Glu Leu
   145                 150                 155                 160
   GCT CAG CTT ACC GCC GAA TTA AAG ATT TAT TCA GTT ATT CAA GCC GAA  528
   Ala Glu Leu Thr Ala Glu Leu Lys Ile Tyr Ser Val Ile Gln Ala Glu
                   165                 170                 175
   ATT AAT AAG CAT CTG TCT AGT AGT GGC ACC ATA AAT ATC CAT GAT AAA  576
   Ile Asn Lys His Leu Ser Ser Ser Gly Thr Ile Asn Ile His Asp Lys
               180                 185                 190
   TCC ATT AAT CTC ATG GAT AAA AAT TTA TAT GGT TAT ACA GAT GAA GAG  624
   Ser Ile Asn Leu Met Asp Lys Asn Leu Tyr Gly Tyr Thr Asp Glu Glu
           195                 200                 205
   ATT TTT AAA GCC AGC GCA GAG TAC AAA ATT CTC GAG AAA ATG CCT CAA  672
   Ile Phe Lys Ala Ser Ala Glu Tyr Lys Ile Leu Glu Lys Met Pro Gln
       210                 215                 220
   ACC ACC ATT CAG GTG GAT GGG AGC GAG AAA AAA ATA GTC TCG ATA AAG  720
   Thr Thr Ile Gln Val Asp Gly Ser Glu Lys Lys Ile Val Ser Ile Lys
   225                 230                 235                 240
   GAC TTT CTT GGA AGT GAG AAT AAA AGA ACC GGG GCG TTG GGT AAT CTG  768
   Asp Phe Leu Gly Ser Glu Asn Lys Arg Thr Gly Ala Leu Gly Asn Leu
                   245                 250                 255
   AAA AAC TCA TAC TCT TAT AAT AAA GAT AAT AAT GAA TTA TCT CAC TTT  816
   Lys Asn Ser Tyr Ser Tyr Asn Lys Asp Asn Asn Glu Leu Ser His Phe
               260                 265                 270
   GCC ACC ACC TGC TCG GAT AAG TCC AGG CCG CTC AAC GAC TTG GTT AGC  864
   Ala Thr Thr Cys Ser Asp Lys Ser Arg Pro Leu Asn Asp Leu Val Ser
           275                 280                 285
   CAA AAA ACA ACT CAG CTG TCT GAT ATT ACA TCA CGT TTT AAT TCA GCT  912
   Gln Lys Thr Thr Gln Leu Ser Asp Ile Thr Ser Arg Phe Asn Ser Ala
       290                 295                 300
   ATT GAA GCA CTG AAC CGT TTC ATT CAG AAA TAT GAT TCA GTG ATG CAA  960
   Ile Glu Ala Leu Asn Arg Phe Ile Gln Lys Tyr Asp Ser Val Met Gln
   305                 310                 315                 320
   CGT CTG CTA GAT GAC ACG TCT GGT AAA TGACACGAGG TAATTATGTA 1007
   Arg Leu Leu Asp Asp Thr Ser Gly Lys
                   325
   AGTCGAC 1014
(2) INFORMATION FOR SEQ ID NO: 4:
   (i) SEQUENCE CHARACTERISTICS:
      (A) LENGTH: 329 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Gly Ile Pro Gly Ile Arg Ala Tyr Glu Gln Asn Pro Gln His Phe Ile
   1 5 10 15
   Glu Asp Leu Glu Lys Val Arg Val Glu Gln Leu Thr Gly His Gly Ser
   20 25 30
   Ser Val Leu Glu Glu Leu Val Gln Leu Val Lys Asp Lys Asn Ile Asp
   35 40 45
   Ile Ser Ile Lys Tyr Asp Pro Arg Lys Asp Ser Glu Val Phe Ala Asn
   50 55 60
   Arg Val Ile Thr Asp Asp Ile Glu Leu Leu Lys Lys Ile Leu Ala Tyr
   65 70 75 80
   Phe Leu Pro Glu Asp Ala Ile Leu Lys Gly Gly His Tyr Asp Asn Gln
   85 90 95
   Leu Gln Asn Gly Ile Lys Arg Val Lys Glu Phe Leu Glu Ser Ser Pro
   100 105 110
   Asn Thr Gln Trp Glu Leu Arg Ala Phe Met Ala Val Met His Phe Ser
   115 120 125
   Leu Thr Ala Asp Arg Ile Asp Asp Asp Ile Leu Lys Val Ile Val Asp
   130 135 140
   Ser Met Asn His His Gly Asp Ala Arg Ser Lys Leu Arg Glu Glu Leu
   145 150 155 160
   Ala Glu Leu Thr Ala Glu Leu Lys Ile Tyr Ser Val Ile Gln Ala Glu
   165 170 175
   Ile Asn Lys His Leu Ser Ser Ser Gly Thr Ile Asn Ile His Asp Lys
   180 185 190
   Ser Ile Asn Leu Met Asp Lys Asn Leu Tyr Gly Tyr Thr Asp Glu Glu
   195 200 205
   Ile Phe Lys Ala Ser Ala Glu Tyr Lys Ile Leu Glu Lys Met Pro Gln
       210                 215                     220
   Thr Thr Ile Gln Val Asp Gly Ser Glu Lys Lys Ile Val Ser Ile Lys
   225                 230                     235                 240
   Asp Phe Leu Gly Ser Glu Asn Lys Arg Thr Gly Ala Leu Gly Asn Leu
                   245                     250                 255
   Lys Asn Ser Tyr Ser Tyr Asn Lys Asp Asn Asn Glu Leu Ser His Phe
               260                 265                     270
   Ala Thr Thr Cys Ser Asp Lys Ser Arg Pro Leu Asn Asp Leu Val Ser
           275                 280                     285
   Gln Lys Thr Thr Gln Leu Ser Asp Ile Thr Ser Arg Phe Asn Ser Ala
       290                 295                     300
   Ile Glu Ala Leu Asn Arg Phe Ile Gln Lys Tyr Asp Ser Val Met Gln
   305                 310                     315                 320
   Arg Leu Leu Asp Asp Thr Ser Gly Lys
                   325

## Description

The present invention relates to novel vaccines for provision of protection against infection with the organism Yersinia pestis and to compositions containing them. Particularly provided are orally active vaccines capable of offering protection against bubonic and pneumonic plague, particularly by induction of mucosal immunity in both humans and other animals.

Yersinia pestis is the highly virulent causative organism of plague in a wide range of animals, including man. Infection with such organisms results in a high rate of mortality. Studies have shown that the high virulence is due to a complex array of factors encoded by both the chromosome and three plasmids, including the Lcr genes (see Straley, 1991), a fibrinolysin (Sodeinde & Goguen, 1988), and a capsule.

Man is an occasional host in the natural cycle of the disease, and bubonic plague, characteried by the swelling of local lymph nodes, may occur following the bite of an infected flea. One of the complications of bubonic plague is secondary pneumonia, and in such cases the disease is readily transmitted between humans by airborne droplets.

Plague is endemic in regions of North and South America, Africa, China and Asia (see Butler (1983) 'Plague and Other Yersinia Infections'; Plenum Press, New York). Current outbreaks are believed to be part of the fourth world pandemic of the disease, and thus there is a clear need to protect individuals living or travelling inn endemic areas, and laboratory workers handling the bacterium.

The current whole cell vaccines available for prevention of plague are highly heterogenous, resulting in side effects which make them unsuitable for widespread use (Reisman, (1970); Meyer et al (1974); Marshall et al (1974)).

One current vaccine for plague is the Cutter vaccine, comprising formaldehyde killed plague bacilli, which is administered to the body by intramuscular injection. However, parenteral immunisation, although effective in inducing systemic immunity, does not effectively induce mucosal immunity (McGhee et al, (1992) Vaccine 10, 75-88). So far no vaccine capable of producing a protective immune response at mucosal surfaces has been reported.

The live attenuated vaccine (Meyer et al ibid) EV76 was tested extensively and used in the former Soviet Union from 1939, although its efficacy in evoking an immune response in man is questionable (Meyer et al (1974) J. Infect. Dis. 129 Supp: 13-18). It has been shown that the virulence of EV76 differs in several animal species, and non-human primates are particularly susceptible to a chronic infection with this strain. In the Western World the vaccine is considered to be unsuitable for mass vaccinations due to the extreme severity of the side effects and the possibility of the strain reverting to full virulence.

One of several known Y.pestis antigens is the Y. pestis LcrV (V antigen), an unstable 37.3 kDa monomeric peptide encoded on the ca. 70 kb Lcr plasmid of Y. pestis, Y. pseudotuberculosis and Y. enterocolitica. This plasmid mediates the growth restriction of the organism at 37°C in the presence of less than 2.5mM Ca²⁺. Under such conditions the cells fail to synthesise bulk vegetative proteins although a series of stress proteins and virulence factors are expressed; this response being known as the 'low calcium response'. A non-polar mutation of the lcrV gene has been shown to cause loss of the requirement for Ca²⁺ and results in avirulence (Price et al (1991) J. Bacteriol 173, pp 2649-2657), thus V antigen is postulated to act as a virulence factor.

Rabbit antiserum rasied against partially purified V antigen has been shown to provide passive protection in mice later challenged intraperitoneally with 100 LD₅₀ Y. pestis (se Lawton et al (1963) J. Immunol 91, pp 179-184). That it might be a virulence factor was confirmed when monospecific antisera raised against Y. pestis V antigen were shown to protect passively against a parenteral challenge with the bacterium (see Une and Brubaker (1984) J. Immunol. 133 pp 2226-2230) and that antibodies raised to a fusion protein of a V-fragment with Protein A provided passive immunity (Une et al (1987) Contrib. Microbiol. Immunol. 9, 179-185).

Recently it was demonstrated that polyclonal antisera raised against recombinant V antigen or a protein A/V or antigen fusion (PAV) were also partially protective against Y.pestis KIM (see Motin et al (1994) Infect. Immun. 62. pp4192-4201). By absorbing the antisera with truncates of PAV, it was deduced that at least one protective epitope lay between amino acids 168 and 275 of V antigen.

The role of V-antigen in virulence is unknown, but Nakajima and Brubaker (1993) Infect. Immun. 61, p23-31 suggested that it may be immunosuppressive, possibly by inhibiting cytokine synthesis, and so prevent the infiltration of host inflammatory cells into infected organs (Une et al (1987) Contrib. Microbiol. Immunol. 9, p179-185; Straley and Cibull (1989) Infect. Immun. 57, p1200-1210). The passive protection conferred by anti-V antigen serum may therefore be attributed to the neutralisation of this immunosuppressive activity (Nakajima and Brubaker (1993) above).

Despite some 30 years having elapsed since the first evidence of its possible implication in Y. pestis virulence there has been no report of use a V antigen based vaccine, whether suitable for oral or parenteral administration or for the purpose of providing mucosal immunity.

The present inventors have now provided recombinant DNA constructs that when incorporated into the DNA of microorganisms, particularly of a human or animal gut colonising microorganism, are capable of transforming it such that it is enabled to express a peptide derived from V antigen, or the V antigen itself, which produces a protective immune response against Yersinia pestis in the human or animal body when the microorganism is administered by oral routes. Preferably the present invention provides such DNA constructs that transform such a microorganism while allowing it maintaining its ability to colonise the human or animal gut and systemically invade the body.

Further provided are plasmids containing these constructs that are capable of transforming a human or animal gut colonising microorganism such that it is enabled to express a protein which produces a protective immune response against Yersinia pestis in a human or animal body when the microorganism is administered by oral routes, and again these preferably allow the microorganism to maintain its ability to colonise the human or animal gut and systemically invade the body.

Still further provided are human or animal gut colonising microorganism transformed with recombinant DNA or a plasmid containing recombinant DNA according to the invention such that it is enabled to express a protein which produces a protective immune response against Yersinia pestis in a human or animal body when the microorganism is administered by oral routes, and preferably capable of maintaining its ability to colonise the human or animal gut. Protective response preferably includes such at mucosal surfaces.

A particularly preferred recombinant DNA, plasmid or human or animal gut colonising organism encodes for or expresses all or a protective epitopic part of the mature V protein of Yersinia pestis. A particularly preferred recombinant DNA comprises a DNA sequence as described in SEQ ID No 1 or SEQ ID No 3, more preferably positioned in frame with a promoter such as lacz or nirβ, and preferably in a vector capable of expression and replication in a Salmonella.

The preferred constructs of the invention allow production of microorganisms that when orally administered induce local stimulation of the gut-associated lymphoid tissue (GALT) and, by trafficking of lymphocytes through the common mucosal immune system provide a secondary stimulation of bronchial associated lymphoid tissue (BALT) thus providing secretory IgA response at respiratory mucosal surfaces.

The microorganisms provided by transformation using the DNA of the invention, in vector or directly inserted format, are preferably attenuated, more preferably attenuated salmonella. Attenuated microorganisms such as S. typhimurium have been well characterised as carriers for various heterologous antigens (Curtiss, (1990); Cardenas and Clements, (1992)). Attenuation may be effected in a number of ways, such as by use of the aro A and/or aro C mutation approach (see Hosieth et al (1981) Nature 291. 238-239; Dougan et al (1986) Parasite Immunol 9, 151-160; Chatfield et al (1989) Vaccine 7. 495-498); multiple mutations such as aro A and aro C mutants as described by Hone et al (1991) Vaccine 9, pp 810-816 may also be used. However any suitably defective organism that is safe for intended use may be employed.

Many other such attenuated deletions and mutations will be known for these and other microorganisms which will render them suitable for transformation with constructs of the present invention for the purposes of expressing vaccine proteins in the gut and/or gut colonisation in animals to be treated for Y.pestis. For human vaccination vectors containing the constructs of the present invention are placed in attenuated S. typhi and that transformed organism used as active agent for a live oral vaccine.

When the DNA of the invention is used to transform the attenuated microorganism by direct insertion into its DNA this may be by direct integration into a gene. Alternatively when incorporated in the form of a plasmid that expresses V protein or an epitopic fragment thereof this may be such that only the V protein or fragment is expressed or that this is expressed as a fusion peptide with a further protein or peptide fragment. Such further protein or peptide fragment might be such as to promote export of mature protein or peptide through the cell membrane or might be a further Y. pestis antigen.

The lcr gene was cloned from Y. pestis strain KIM by Price et al and its nucleotide sequence published in J. Bacteriol (1989) 171, pp 5646-5653. In the examples below this information was used to design oligonucleotide primers which could amplify the gene from Y. pestis (strain GB) using the polymerase chain reaction (PCR). PCR primers were designed to be complementary to respective sequences flanking the 5' and 3' ends of the IcrV gene but also having 5' end tails including a restriction enzyme recognition site to enable cloning of amplified lcrV gene directionally into a plasmid vector (the 5' primer including an EcoRI site and the 3' primer containing a SacI site).

In the examples below the constructs of the invention include a lac promoter, but other promoters such as the macrophage promoter (nirβ) may be used.

The method, constructs, microorganisms and vaccines of the invention will now be exemplified by way of illustration only by reference to the following Sequence listing, Figure and Examples. Still further embodiments will be evident to those skilled in the art in the light of these.

### SEQUENCE LISTING:

SEQ ID No 1: Shows the nucleotide and derived amino acid sequence of a DNA of the invention with the last 6 bases of vector pMAL-p2 or pMAL-c2 into which it is cloned at the 5' end using the EcoRI site in sequence GAATTC (derived from the 5' end PCR primer) and at the 3' end at the SalI site in sequence GTCGAC (derived from the 3' end PCR primer). The base at position 1006 has been altered by SDM to a T to create a second in frame stop codon. The start of the sequence is a factor Xa cleavage site.

SEQ ID No 2: Shows the amino acid sequence of the peptide expressed by the DNA of the invention, with two amino acids encoded for by the vector (I and S) at the N-terminal end.

SEQ ID No 3: Shows the nucleotide and derived amino acid sequence of a second DNA of the invention with the last 10 bases of a vector pGEX-5X-2 into which it is cloned shown at the 5' end using the EcoRI site in sequence GAATTC (GA derived from the 5' end PCR primer) and the SalI site in sequence GTCGAC (GTCGAC derived from the 3' end PCR primer). The base at position 1006 has been altered by SDM to create a second in frame stop codon; the base at position 16 has been altered to a C from an A to create the EcoRI site. The start of the sequence is a factor Xa cleavage site.

SEQ ID No 4: Shows the amino acid sequence of the peptide expressed by the second DNA of the invention, with four amino acids encoded by the vector (G, I. P and G) at the N-terminal end.

EXAMPLES. Manipulation of DNA. Chromosomal DNA was isolated from Y. pestis by the method of Marmur. The gene encoding V-antigen (1crV) was amplified from Y.pestis DNA using the polymerase chain reaction (PCR) with 125pmol of primers homologous to sequences from the 5' and 3' ends of the gene (see Price et al (1989) J. Bacteriol 171 p5646-5653).

The sequences of the 5' primer (V/5'E: GATCGAATTCATTAGAGCCTACGAACAA) and the 3' primer (GGATCGTCGACTTACATAATTACCTCGTGTCA) also included 5' regions encoding the restriction sites EcoRl and Sall, respectively. In addition, one nucleotide (*) was altered from the published sequence of IcrV (Price et al, 1989), so that the amplified gene encoded an extra termination codon (TAA). The PCR primers were prepared with a DNA synthesiser (392 Applied Biosystems). A DNA fragment was obtained after 30 cycles of amplification (95°C. 20secs, 45°C, 20secs, 72°C,. 30 secs; Perkin 9600 GeneAmp PCR System). The fragment was purified, digested with EcoRI and SalI, ligated with suitably digested plasmid pGEX-5X-2

Amplified lcrV gene was cloned into three different plasmid vectors:

### EXAMPLE 1:

pMAL-p2: a vector designed to express the cloned gene as a fusion product with a maltose binding protein (MBP). The C-terminus of the MBP is fused to the N-terminus of the V-antigen. The fusion protein so produced on expression is exported to the periplasm. Vector including the V-antigen DNA sequence was designated pVMP100.

### EXAMPLE 2:

pMAL-c2: a vector similar to pMAL-p2 except that MBP-V antigen fusion protein is expressed cytoplasmically. The recombinant plasmid was designated pVMC100.

### EXAMPLE 3:

pGEX-5X-2: a vector designed to express the cloned gene as a fusion protein with glutathione-S-transferase (GST). The C-terminus of GST is fused to the N-terminus of V antigen and the fusion protein is expressed cytoplasmically. The recombinant plasmid was designated pVG100.

All the vectors contain the P_{tac} promoter and the lacI^{Q} gene; the latter encoding the lac repressor which turns off transcription from P_{tac} in Escherichia coli until IPTG is added. The plasmids contain the origin of replication from pBR322 and as a result replicate to a low copy number in the bacterial cell. Each of the recombinant plasmids were electroporated into Salmonella typhimurium strain SL3261, an attenuated strain that has been used extensively as a live vaccine vector for the expression of foreign antigens. It contains a specific deletion mutation in the aroA gene which makes the mutant dependent upon certain aromatics for growth (see Hosieth et al). For producing microorganism suitable for human vaccination use electroporation is into attenuated Salmonella typhi.

The recombinant plasmids all expressed V antigen as shown by Western blotting of S. typhimurium cultures and probing with a monospecific anti-V antigen polyclonal antiserum supplied by R Brubaker. Dept Microbiology, Michigan State University, East Lansing, MI 48824-1101, USA. Recombinant S. typhimurium were innoculated intravenously into mice at 5 x 10⁷ cfu/dose and shown to colonise the liver and spleen at high levels; between 8 x 10⁶ and 5 x 10⁸ cfu per organ were recovered. The majority of the bacterial cells recovered were also ampicillin resistant indicating retention of recombinant plasmids.

## Claims

1. The use of a recombinant microorganism which has been transformed so that it is enabled to express a nucleic acid sequence which encodes the V-antigen of Yersinia pestis or an immunogenic fragment or derivative thereof which is able to produce a protective immune response against Yersinia pestis in a human or animal body, in the preparation of an oral vaccine for protection against infection by Yersinia pestis.

2. The use according to claim 1 wherein the microorganism is able to colonise the human or animal gut.

3. The use according to claim 1 or claim 2 wherein the microorganism is able to invade the human body systemically.

4. The use according to any one of claims 1 to 3 wherein the microorganism is an attenuated microorganism.

5. The use according to claim 4 wherein the microorganism is an Aro A or Aro C mutant.

6. The use according to any one of claims 1 to 5 wherein the microorganism comprises a Salmonella.

7. The use according to claim 6 wherein the Salmonella is Salmonella typhimurium or a Salmonella typhi.

8. The use according to any one of the preceding claims wherein the said nucleic acid encodes a fusion peptide comprising the V-antigen of Yersinia pestis or an immunogenic fragment or derivative thereof which is able to produce a protective immune response against Yersinia pestis in a human or animal body, fused to a peptide or protein which is either able to promote export of mature protein or peptide through a cell membrane, or a further Yersinia pestis antigen.

9. The use according to claim 8 wherein the said peptide or protein is either maltose binding protein or glutathione-S-transferase.

10. The use according to claim 1 wherein the said nucleic acid encodes an amino acid sequence comprising SEQ ID NO 2 or SEQ ID NO 4.

11. The use according to claim 1 wherein the said nucleic acid is under the control of a lac promoter or a nirβ promoter.

12. The use of a recombinant nucleic acid sequence which encodes the V-antigen of Yersinia pestis or an immunogenic fragment or derivative thereof which is able to produce a protective immune response against Yersinia pestis in a human or animal body, in the preparation of an oral vaccine for protection against infection by Yersinia pestis.

13. The use according to claim 12 wherein the nucleic acid sequence encodes a fusion peptide comprising the V-antigen of Yersinia pestis or an immunogenic fragment or derivative thereof which is able to produce a protective immune response against Yersinia pestis in a human or animal body, fused to a peptide or protein which is either able to promote export of mature protein or peptide through a cell membrane, or a further Yersinia pestis antigen.

14. The use according to claim 13 wherein the said peptide or protein is either maltose binding protein or glutathione-S-transferase.

15. A vaccine comprising a recombinant microorganism which has been transformed so that it is enabled to express a nucleic acid sequence which encodes the V-antigen of Yersinia pestis or an immunogenic fragment or derivative thereof which is able to produce a protective immune response against Yersinia pestis in a human or animal body, in combination with a pharmaceutically acceptable carrier, which vaccine is adapted for oral administration.

## Patentansprüche

1. Verwendung eines rekombinerten Mikroorganismus, der so transformiert worden ist, dass er befähigt ist, eine Nucleinsäuresequenz zu exprimieren, die das V-Antigen von *Yersinia pestis* oder ein immunogenes Fragment oder Derivat davon codiert, das imstande ist, eine schützende Immunantwort gegen *Yersinia pestis* in einem menschlichen Körper oder einem Tierkörper hervorzurufen, zur Herstellung eines oralen Impfstoffs für den Schutz vor einer Infektion mit *Yersinia pestis*.

2. Verwendung nach Anspruch 1, wobei der Mikroorganismus imstande ist, den Darm des Menschen oder von Tieren zu besiedeln.

3. Verwendung nach Anspruch 1 oder Anspruch 2, wobei der Mikroorganismus imstande ist, systemisch in den menschlichen Körper einzudringen.

4. Verwendung nach einem der Ansprüche 1 bis 3, wobei der Mikroorganismus ein abgeschwächter Mikroorganismus ist.

5. Verwendung nach Anspruch 4, wobei der Mikroorganismus eine Aro-A- oder Aro-C-Mutante ist.

6. Verwendung nach einem der Ansprüche 1 bis 5, wobei der Mikroorganismus eine *Salmonella* umfasst.

7. Verwendung nach Anspruch 6, wobei es sich bei der *Salmonella* um *Salmonella typhimurium* oder eine *Salmonella typhi* handelt.

8. Verwendung nach einem der vorhergehenden Ansprüche, wobei die Nucleinsäure ein Fusionspeptid codiert, das das V-Antigen von *Yersinia pestis* oder ein immunogenes Fragment oder Derivat davon umfasst, das imstande ist, eine schützende Immunantwort gegen *Yersinia pestis* in einem menschlichen Körper oder einem Tierkörper hervorzurufen, das mit einem Peptid oder Protein verknüpft ist, das befähigt ist, den Export von reifem Protein oder Peptid durch eine Zellmembran zu fördern, oder das ein weiteres *Yersinia pestis*-Antigen ist.

9. Verwendung nach Anspruch 8, wobei es sich bei dem Peptid oder Protein um Maltose-bindendes Protein oder Glutathion-S-Transferase handelt.

10. Verwendung nach Anspruch 1, wobei die Nucleinsäure eine Aminosäuresequenz codiert, die SEQ ID NO 2 oder SEQ ID NO 4 umfasst.

11. Verwendung nach Anspruch 1, wobei die Nucleinsäure unter der Kontrolle eines lac-Promotors oder eines nirβ-Promotors steht.

12. Verwendung einer rekombinanten Nucleinsäuresequenz, die das V-Antigen von *Yersinia pestis* oder ein immunogenes Fragment oder Derivat davon codiert, das befähigt ist, eine schützende Immunantwort gegen *Yersinia pestis* in einem menschlichen Körper oder einem Tierkörper hervorzurufen, zur Herstellung eines oralen Impfstoffs für den Schutz vor einer Infektion mit *Yersinia pestis*.

13. Verwendung nach Anspruch 12, wobei die Nucleinsäuresequenz ein Fusionspeptid codiert, das das V-Antigen von *Yersinia pestis* oder ein immunogenes Fragment oder Derivat davon umfasst, das imstande ist, eine schützende Immunantwort gegen *Yersinia pestis* in einem menschlichen Körper oder einem Tierkörper hervorzurufen, das mit einem Peptid oder Protein verknüpft ist, das befähigt ist, den Export von reifem Protein oder Peptid durch eine Zellmembran zu fördern, oder das ein weiteres *Yersinia pestis*-Antigen ist.

14. Verwendung nach Anspruch 13, wobei es sich bei dem Peptid oder Protein um Maltose-bindendes Protein oder Glutathion-S-Transferase handelt.

15. Impfstoff, der einen rekombinanten Mikroorganismus enthält, der so transformiert worden ist, dass er befähigt ist, eine Nucleinsäuresequenz zu exprimieren, die das V-Antigen von *Yersinia pestis* oder ein immunogenes Fragment oder Derivat davon codiert, das imstande ist, eine schützende Immunantwort gegen *Yersinia pestis* in einem menschlichen Körper oder einem Tierkörper hervorzurufen, in Kombination mit einem pharmazeutisch akzeptablen Träger, wobei der Impfstoff an eine orale Verabreichung angepasst ist.

## Revendications

1. Utilisation d'un micro-organisme recombinant qui a été transformé de telle sorte qu'il lui soit possible d'exprimer une séquence d'acide nucléique qui code pour l'antigène V de *Yersinia pestis* ou un fragment immunogène ou un dérivé de celui-ci qui est capable d'induire une réponse immunitaire protectrice contre *Yersinia pestis* dans un corps humain ou animal, dans la préparation d'un vaccin oral pour la protection contre l'infection par *Yersinia pestis.*

2. Utilisation selon la revendication 1, dans laquelle le micro-organisme est capable de coloniser l'intestin humain ou animal.

3. Utilisation selon la revendication 1 ou la revendication 2, dans laquelle le micro-organisme est capable d'envahir systémiquement le corps humain.

4. Utilisation selon l'une quelconque des revendications 1 à 3, dans laquelle le micro-organisme est un micro-organisme atténué.

5. Utilisation selon la revendication 4, dans laquelle le micro-organisme est un mutant Aro A ou Aro C.

6. Utilisation selon l'une quelconque des revendications 1 à 5, dans laquelle le micro-organisme comprend une *Salmonella.*

7. Utilisation selon la revendication 6, dans laquelle la *Salmonella* est la *Salmonella typhimurium* ou une *Salmonella typhi*.

8. Utilisation selon l'une quelconque des revendications précédentes, dans laquelle ledit acide nucléique code pour un peptide de fusion comprenant l'antigène V de *Yersinia pestis* ou un fragment immunogène ou un dérivé de celui-ci qui est capable d'induire une réponse immunitaire protectrice contre *Yersinia pestis* dans un corps humain ou animal, fusionné à un peptide ou à une protéine qui est soit capable de promouvoir l'exportation de la protéine mature ou du peptide au travers de la membrane cellulaire, soit un autre antigène de *Yersinia pestis*.

9. Utilisation selon la revendication 8, dans laquelle ledit peptide ou ladite protéine est soit une protéine de liaison au maltose soit la glutathion-S-transférase.

10. Utilisation selon la revendication 1, dans laquelle ledit acide nucléique code pour une séquence d'acides aminés comprenant la SEQ ID NO : 2 ou la SEQ ID NO : 4.

11. Utilisation selon la revendication 1, dans laquelle ledit acide nucléique se trouve sous le contrôle d'un promoteur *lac* ou d'un promoteur *nir*β.

12. Utilisation d'une séquence d'acide nucléique recombinant qui code pour l'antigène V de *Yersinia pestis* ou un fragment immunogène ou un dérivé de celui-ci qui est capable d'induire une réponse immunitaire protectrice contre *Yersinia pestis* dans un corps humain ou animal, dans la préparation d'un vaccin oral pour la protection contre l'infection par *Yersinia pestis.*

13. Utilisation selon la revendication 12, dans laquelle la séquence d'acide nucléique code pour un peptide de fusion comprenant l'antigène V de *Yersinia pestis* ou un fragment immunogène ou un dérivé de celui-ci qui est capable d'induire une réponse immunitaire protectrice contre *Yersinia pestis* dans un corps humain ou animal, fusionné à un peptide ou à une protéine qui est soit capable de promouvoir l'exportation de la protéine mature ou du peptide au travers de la membrane cellulaire, soit un autre antigène de *Yersinia pestis*.

14. Utilisation selon la revendication 13, dans laquelle ledit peptide ou ladite protéine est soit une protéine de liaison au maltose soit la glutathion-S-transférase.

15. Vaccin comprenant un micro-organisme recombinant qui a été transformé de telle sorte qu'il lui soit possible d'exprimer une séquence d'acide nucléique qui code pour l'antigène V de *Yersinia pestis* ou un fragment immunogène ou un dérivé de celui-ci qui est capable d'induire une réponse immunitaire protectrice contre *Yersinia pestis* dans un corps humain ou animal, en combinaison à un support pharmaceutiquement acceptable, lequel vaccin est adapté à l'administration par voie orale.
